# FASCICULE DE BREVET EUROPEEN

(11) **EP 1 613 375 B1**
(45) Date de publication et mention de la délivrance du brevet: **04.10.2006**
(21) Numéro de dépôt: 04742467.6
(22) Date de dépôt: 09.04.2004
(51) Int. Cl.: A61M 5/30

(54) **SERINGUE SANS AIGUILLE AVEC UN INJECTEUR-RECEPTACLE**
NADELLOSE SPRITZE MIT EINEM INJEKTOREN-AUFNAHMEBEHÄLTER
NEEDLELESS SYRINGE COMPRISING AN OPTIMIZED INJECTOR-RECEPTACLE

(30) Priorité: 16.04.2003 FR 0304761
(43) Date de publication de la demande: 11.01.2006
(73) Titulaire: Crossject, 75004 Paris (FR)
(72) Inventeur: ALEXANDRE, Patrick, F-70100 Gray (FR); BAUD, Georges, F-83260 La Crau (FR); BROUQUIERES, Bernard, F-83100 Toulon (FR)
(74) Mandataire: Maureau, Philippe
(86) Numéro de dépôt international: PCT/FR2004/000881
(87) Numéro de publication internationale: WO 2004/093944

(56) Documents cités:
- EP-A- 0 896 827
- WO-A-00/48654
- WO-A-01/58512
- US-A- 4 668 223

## Description

La présente invention est dans le domaine des seringues sans aiguille jetables ; de telles seringues sont utilisées pour les injections intradermiques, sous-cutanées et intramusculaires, de principe actif liquide à usage thérapeutique en médecine humaine ou vétérinaire.

Un premier impératif pour des seringues sans aiguille est celui de la compatibilité à long terme, entre le principe actif liquide et le réservoir qui le contient. Un autre impératif est d'avoir un réservoir transparent pour faire les contrôles du remplissage correct du réservoir avant l'utilisation de la seringue. Ces impératifs conduisent à la réalisation de réservoir essentiellement transparent et en matériau compatible avec le principe actif pour la durée souhaitée : c'est en général du verre borosilate à usage pharmaceutique : verre de type I ou II.

La phase initiale de l'injection est critique pour la pénétration dans la peau du jet ou des jets de liquide, suivant que la seringue a un ou plusieurs conduits d'injection. Cette dernière configuration étant favorable pour réduire la douleur. La biodisponibilité finale dépend de la bonne réalisation de cette phase initiale, elle suppose une mise en vitesse rapide du liquide dans les conduits d'injection sans les saccades multiples des jets quand il y a un coup de bélier trop important pour réaliser cette mise en vitesse rapide.

La demande de brevet WO 01/58512 décrit une seringue sans aiguille comportant un réservoir fermé par des obturateurs amont et aval déplaçables emprisonnant un principe actif liquide ; ledit réservoir est initialement isolé d'un injecteur ou réceptacle qui comporte au moins deux conduits d'injection situés sur sa face latérale extérieure et un alésage central borgne dans lequel va venir se loger l'obturateur aval de façon à dégager les entrées des conduits d'injection lors du déplacement de l'ensemble obturateur aval - principe actif - obturateur amont par l'action d'un dispositif moteur pour réaliser l'injection.

Une première difficulté pour cette seringue est la non optimisation des entrées dans les conduits d'injection. Non optimisation d'une part vis-à-vis des pertes de charges et d'autre part vis-à-vis des pertes de liquide résiduel dans les volumes morts. Dans cette demande les entrées sont des rainures radiales sur toute la face amont du réceptacle. Enfin la forme de l'ouverture amont de l'alésage central n'est pas optimisée et peut déchirer l'obturateur aval lorsqu'il s'y engage.

Un problème important n'est pas résolu de façon satisfaisante dans cette invention, c'est celui de la minimisation des efforts de pression sur le réceptacle pendant la phase d'injection sachant que ces efforts sont dimensionnants pour le réceptacle. Compte tenu du montage réalisé, toute la surface du réceptacle en appui sur le réservoir est soumise à la pression car l'étanchéité est reportée vers le matériau de frettage à l'extérieur du réservoir car l'utilisation d'un joint torique est ici impossible notamment à cause des rainures radiales d'entrées dans les conduits d'injection.

La présente invention vise à résoudre les problèmes d'optimisation des entrées dans les conduits d'injection et surtout ceux de réduction des efforts de pression sur le réceptacle pendant la phase d'injection.

L'invention est plus particulièrement définie dans la revendication indépendante et les revendications dépendantes annexée à la présente description.

Dans cette demande est désignée avec le qualificatif aval toute pièce proche du site d'injection ou toute partie de pièce dirigée vers ce site d'injection, ce site est la peau du patient. A contrario le qualificatif amont sera utilisé pour toute pièce éloignée du site d'injection ou toute partie de pièce dirigée à l'opposé de ce site. Ainsi le réceptacle comporte une face aval dirigée vers la peau du patient et une face amont qui lui est opposée et est en appui sur le réservoir ; ces faces aval et amont sont reliées par une face latérale.

Dans cette invention par principe actif liquide, ou médicament, nous entendrons essentiellement un liquide plus ou moins visqueux, ou un mélange de liquides, ou un gel. Le principe actif pourra être un solide mis en solution dans un solvant approprié pour l'injection. Le principe actif pourra être un solide sous forme pulvérulente mis en suspension, plus ou moins concentrée, dans un liquide approprié. La granulométrie du principe actif solide doit être adaptée, ainsi que la forme du conduit pour éviter les bouchages.

Le réservoir, essentiellement cylindrique, est en verre de type I ou de type II ; mais il peut être en tout autre matériau transparent et compatible avec le principe actif. Les faces amont et aval du réservoir sont essentiellement planes, les plans les contenant étant perpendiculaires à l'axe de symétrie du réservoir. Les faces amont et aval sont en appui respectivement avec le corps de la seringue et le réceptacle. Les faces d'appui de ces deux pièces comportent des joints dont les caractéristiques seront précisées par la suite.

Un conduit d'injection traverse toute la hauteur du réceptacle depuis la face amont jusqu'à la face aval. Les conduits d'injection, puisqu'il y en a au moins deux, sont dits périphériques car ils sont disposés dans le réceptacle autour de l'alésage central borgne. Ils ne communiquent avec ledit alésage central que par des entrées décrites par la suite. Le conduit d'injection a une section variable de l'amont à l'aval d'une part pour des raisons liées à sa réalisation et d'autre part pour obtenir un jet suffisamment fin et rapide pour pénétrer, à la profondeur souhaitée, dans la peau du patient. En général les conduits d'injection sont identiques, équirépartis autour de l'alésage central borgne et ont des axes parallèles à l'axe de réceptacle, mais ils peuvent aussi être différents.

Le moyen moteur qui va agir sur l'obturateur amont peut être un moteur mécanique : détente d'un ressort comprimé ou du type pneumatique : détente de gaz comprimé, ou pyrotechnique : détente de gaz de combustion.

Le fonctionnement de la seringue est le suivant : le moyen moteur va agir sur l'obturateur amont et déplacer l'ensemble obturateur amont - liquide - obturateur aval puisque le liquide est incompressible. L'obturateur aval se déplace et se loge dans l'alésage borgne du réceptacle jusqu'au contact avec le fond dudit alésage. La hauteur de cet alésage est telle que lorsque l'obturateur aval est au contact du fond dudit alésage les entrées des conduits d'injection, sur la périphérie de l'alésage du réceptacle, sont dégagées ; le liquide y est refoulé et est injecté par le mouvement de l'obturateur amont qui se poursuit jusqu'à la vidange du réservoir : l'obturateur amont est alors au contact de l'obturateur aval.

L'entrée d'un conduit d'injection, située sur la face amont du réceptacle comprend un lamage positionné et préférentiellement centré sur le conduit d'injection et un canal radial reliant ledit lamage à l'alésage central borgne dudit réceptacle.
Le lamage formant l'entrée du conduit d'injection a un diamètre compris entre environ 1,1 fois et environ 1,5 fois le diamètre du conduit d'injection, et préférentiellement environ 1,2 fois ce diamètre. Le diamètre du conduit pris ici pour référence est celui près de la face amont du réceptacle. La profondeur du lamage est comprise entre environ 0,5 fois et environ 0,7 fois le diamètre du conduit d'injection et préféren-tiellement environ 0,6 fois ce diamètre.
Un canal radial, se raccordant au lamage précédemment décrit, relie l'alésage au conduit d'injection. La profondeur dudit canal radial est égale à celle du lamage. La largeur de ce canal radial est soit constante et égale au diamètre du lamage soit cette largeur va croître depuis celle qu'elle a à son raccor-dement au lamage jusqu'à une valeur supérieure à son débouché dans l'alésage central : cette valeur supérieure restera cependant inférieure à environ 1,4 fois le diamètre du conduit d'injection.
Le lamage peut être pris dans son sens technique habituel : c'est-à-dire comme un usinage en forme d'évidemment circulaire dressé exécuté autour d'un trou, dans ce cas la face latérale du lamage est perpen-diculaire à la face amont plane du réceptacle et au fond du lamage.

Mais on peut entendre aussi lamage dans un sens plus large où la face latérale de l'évidemment ne serait plus perpendiculaire mais oblique par rapport à la face amont et même raccordé au fond de l'évidemment par un arrondi. Dans ce cas les dimensions du lamage et du canal radial seront prises à leur partie amont.

La face amont du réceptacle comporte un orifice central constitué par l'alésage central borgne et les entrées reliant ledit alésage aux conduits d'injection. Les bords de cet orifice sont les intersections de la face amont plane et des faces latérales de l'alésage et des entrées. Théoriquement ces bords correspondent à des arêtes plus ou moins vives, mais ces arêtes sont émoussées avec un rayon de courbure convenable surtout sur les portions qui sont les bords de l'alésage central et ceux de l'intersection des canaux radiaux pour ne pas blesser ni déchirer l'obturateur aval quand il s'engage dans l'alésage central en début de fonctionnement et qu'il passe "sur" ses portions.
Cette face amont comporte un joint d'étanchéité multilobé parallèle et au plus près des bords de cet orifice central. Le joint est dit parallèle aux bords car sa distance au bord, évaluée suivant une direction perpendiculaire au bord est constante, le joint est dit au plus près du bord car cette distance est la plus petite possible compte tenu des modes de réalisation du réceptacle. Le joint est multilobé car il contourne par l'extérieur, les entrées des conduits d'injection en étant parallèle et au plus près du bord ; ici la notion d'extérieur étant évaluée par l'éloignement par rapport à l'axe.

Par rapport à un joint circulaire qui entourerait l'alésage et les entrées des conduits, le joint multilobé tel que défini, réduit la surface du réceptacle exposée à la pression du liquide pendant la phase d'injection, car pour les secteurs entre les conduits le joint multilobé est plus près du bord de l'alésage qu'un joint circulaire.

Selon une première réalisation le joint multilobé est un joint rapporté, logé dans une gorge de forme conjuguée réalisée sur la face amont du réceptacle. Cette réalisation implique la mise en place du joint dans la gorge par des procédés précis puis des manipulations minutieuses pour le montage en appui sur le réservoir, puis dans le corps de la seringue.

Selon une deuxième réalisation préférée, lorsque le réceptacle est réalisé par injection, le joint multilobé tel que précédemment décrit est réalisé par biinjection. Le joint multilobé et biinjecté comporte une surépaisseur centrale sur sa face extérieure ; la compression et la déformation de cette surépaisseur lors de la mise en appui du réceptacle et du réservoir assure l'étanchéité.
La technique de biinjection, connue par ailleurs, consiste lorsque la pièce principale, ici le réceptacle, a été injectée et n'est pas encore durcie à la reprendre et à injecter avec un moule approprié le matériau qui réalisera le joint multilobé dans l'empreinte prévue à cet effet dans le réceptacle. Cette empreinte doit bien sûr être parallèle aux bords de l'orifice et au plus près du bord. On assimilera à cette technique celle du surmoulage du joint multilobé.
Le joint multilobé biinjecté présente l'avantage d'obtenir quasi directement après cette deuxième injection une pièce unique réceptable-joint pour continuer ensuite le montage de la seringue.

Avantageusement l'alésage central du réceptacle est de forme essentiellement tronconique, le diamètre d'entrée dudit alésage étant égal au diamètre intérieur du réservoir et le plus petit diamètre de l'alésage étant vers le fond dudit alésage, une génératrice courante de la partie latérale faisant un angle compris entre environ 2° et environ 9° avec l'axe de symétrie de l'alésage et préférentiellement cet angle est d'environ 7°. Le fond de l'alésage est raccordé à la partie latérale par un arrondi convenable.
La profondeur de l'alésage est telle que lorsque l'obturateur aval est en appui sur le fond de l'alésage les entrées des conduits d'injection sont dégagées et mettent le réservoir en communication avec les conduits d'injection. La forme relativement ouverte de l'alésage fait que l'obturateur aval s'engage dans cette cavité et se déforme régulièrement pendant cette phase ce qui amortit le choc et limite les efforts sur le réceptacle lors de cette phase du fonctionnement.

La présente invention appliquée à une seringue pré-remplie et à usage unique a l'avantage de permettre de séparer, dans le dispositif, deux parties. Une partie qui sera dite partie pharmaceutique comprenant le corps et le réservoir avec les obturateurs déplaçables amont et aval et éventuellement l'injecteur-réceptacle : ce sous-ensemble pourra être traité dans les conditions de l'industrie pharmaceutique notamment en ce qui concerne la stérilisation et l'asepsie. Ce sous-ensemble sera intégré au reste de la seringue, dont les éléments ont été assemblés par ailleurs, cet assemblage se faisant dans les conditions moins sévères que celles liées à l'industrie pharmaceutique.

Lorsque l'obturateur aval est logé dans l'alésage du réceptacle la seringue devient très difficilement réutilisable. Cette disposition a donc aussi l'avantage d'empêcher des réutilisations de ladite seringue à des fins différentes de l'utilisation thérapeutique initiale.

Enfin cette configuration présente l'avantage d'éviter des fuites éventuelles de liquide par les conduits d'injection avant la réalisation de l'injection. En effet l'agitation du dispositif est fréquemment réalisée, voire préconisée pour examiner la turbidité du liquide ou homogénéiser le mélange lorsque le liquide comporte des particules en suspension. Le fait que le principe actif soit isolé, avant injection, des conduits réalisent une protection ultime vis à vis de ce risque de perte.

Ci-dessous l'invention est exposée en détail à l'aide de figures représentant différentes réalisations particulières de l'invention.

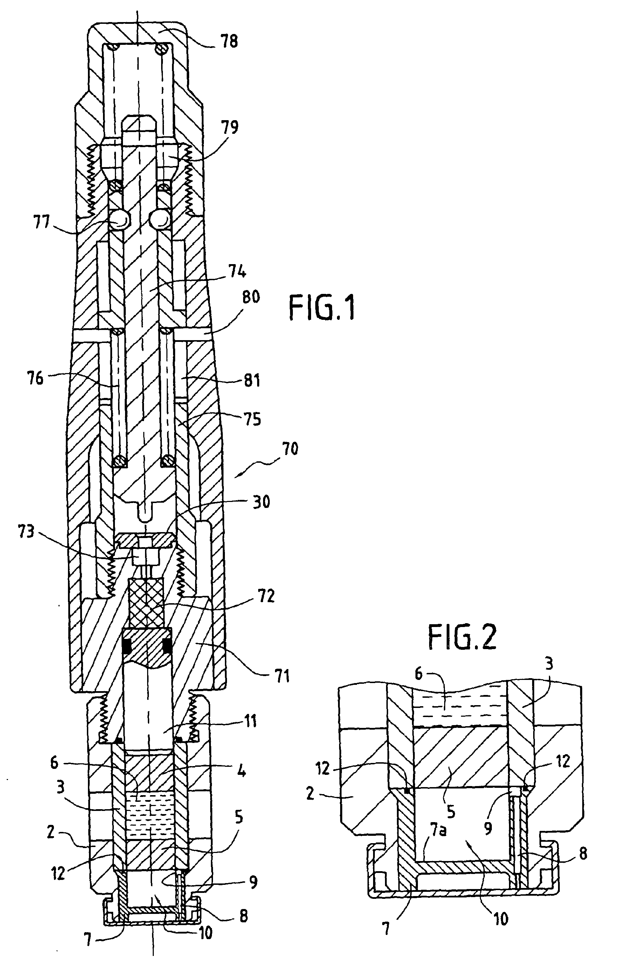

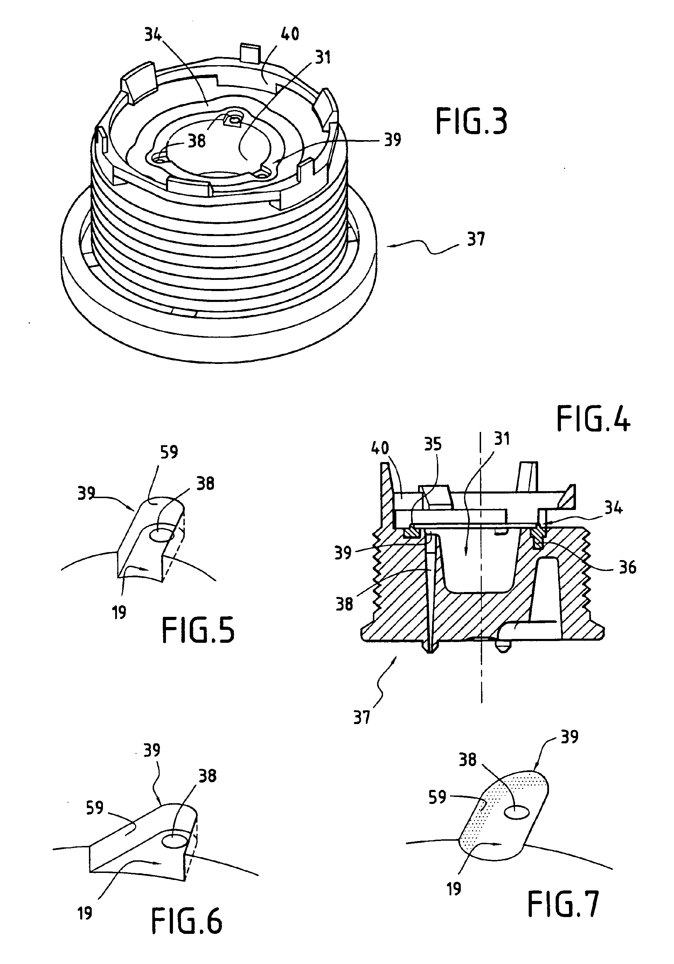

La figure 1 représente une coupe longitudinale d'une seringue selon une première réalisation de l'invention. La figure 2 est un agrandissement de la partie aval de ladite seringue. La figure 3 est une vue en perspective d'un autre exemple de réalisation du réceptacle selon l'invention. La figure 4 représente une coupe dudit réceptacle par un plan passant par l'axe central du réceptacle et contenant l'axe d'un conduit d'injection. Les figures 5, 6 et 7 représentent schématiquement et en perspective différentes formes d'entrées dans les conduits d'injection.

La figure 1 représente en coupe longitudinale partielle une seringue selon l'invention, elle est représentée verticale, le système d'injection dirigé vers le bas qui sera l'aval.

La seringue 1 comporte un corps 2 dans lequel est logé un réservoir 3 contenant le principe actif liquide 6. A l'extrémité aval du corps 2 est placé un réceptacle 7 comportant, par exemple, trois conduits d'injection tels que le conduit 8. Le système d'injection est recouvert d'une protection extérieure pour assurer l'asepsie de la seringue : cette protection comprend une membrane d'élastomère appliquée sur la face extérieure de l'injecteur par un opercule métallique fin, serti autour de cette extrémité de la seringue. Cette protection sera retirée avant l'injection. A son extrémité opposée, le corps 2 de la seringue est fixé à un moyen moteur 70 qui, dans cet exemple, est un générateur pyrotechnique de gaz, il sera décrit par la suite. Le réservoir 3 est en appui sur le corps 71 du moteur 70, l'étanchéité est assurée par un joint torique circulaire.

Le corps 2 de la seringue comporte deux fenêtres diamétralement opposées pour la visualisation du principe actif contenu dans le réservoir 3 : ce sont simplement deux ouvertures oblongues dans le corps. A l'aval du corps 2 de la seringue est emmanché, dans un alésage de forme appropriée, un réceptacle 7 cylindro-conique qui sera décrit par la suite. En appui sur ce réceptacle 7 et centré dans l'aval du corps 2 est positionné un réservoir 3 de verre ; ce réservoir est un tube. En amont le corps 2 de la seringue reçoit le corps 71 du moyen moteur qui se centre autour de l'autre extrémité du réservoir. Le réservoir 3 est essentiellement un tube fermé à ses deux extrémités par des obturateurs déplaçables amont 4 et aval 5 ; ces obturateurs sont préférentiellement des bouchons-pistons habituellement utilisés dans les seringues : ce sont des pièces obtenues par moulage d'élastomères compatibles pour une longue durée avec le principe actif : chaque pièce intègre les fonctions de piston et d'étanchéité par la réalisation de bourrelets ou de lèvres (non détaillée sur les figures). Les élastomères habituellement utilisés pour la fabrication de ces pièces sont par exemple des chlorobutyl ou bromobutyl, dont la dureté Shore est réglée entre environ 45 et environ 70. Ces pièces peuvent recevoir des traitements de surface notamment pour faciliter leurs déplacements dans le réservoir tubulaire. Lorsqu'il est libre, le bouchon-piston a un diamètre supérieur d'environ 10 pour cent au diamètre intérieur du tube qui va le recevoir, la hauteur du bouchon-piston est d'environ 0,5 à 0,8 fois ce diamètre. Lorsque le bouchon-piston est engagé dans le tube, du fait des déformations, sa hauteur est égale à environ 0,6 fois à environ 1,0 fois le diamètre intérieur du réservoir.

Le réceptacle 7 est dans cet exemple une pièce de forme extérieure cylindro-conique qui comporte un alésage central 10 dans lequel va venir se loger l'obturateur aval 5. Sur sa périphérie le réceptacle comporte trois conduits d'injection dont un seul, repéré 8, est visible sur cette coupe. Le diamètre de l'alésage est égal à celui du réservoir. La hauteur libre de l'alésage borgne 10 du réceptacle 7 est égale à celle dé l'obturateur aval 5 monté dans le réservoir 3. Lorsque l'obturateur aval 5 a atteint le fond 7a du réceptacle, l'entrée 9 (côté réservoir 3) des conduits d'injection 8 est mise en communication avec le liquide 6 ; le liquide s'écoule avec une vitesse correspondant à la pression transmise par l'obturateur amont 4.

Dans cette réalisation le moyen moteur agit sur l'obturateur amont par l'intermédiaire d'un piston 11 de section efficace égale à celle de l'obturateur amont 4. Ce piston 11 est en contact avec l'obturateur amont 4 il n'y a donc pas d'effet de choc ou de bélier en début de fonctionnement. Ce piston 11 grâce à son système d'étanchéité empêche les gaz produits par la combustion du chargement 72 de venir en contact avec l'obturateur amont et donc d'éventuelles détériorations de célui-ci et des fuites de gaz vers le principe actif contenu dans le réservoir. Ce piston 11, d'une couleur adaptée, peut aussi servir d'indicateur de fonctionnement en apparaissant dans les fenêtres de visualisation du corps 2 de la seringue.

Nous allons décrire les principaux éléments, du générateur pyrotechniques 70. Il comprend dans le corps 71 au-dessus du piston un chargement pyrotechnique 72 dont la combustion est initiée par une amorce 73 impactée par un percuteur 74. L'amorce 73 est logée dans un porte-amorce. En position initiale le percuteur 74 est retenu, dans le guide-percuteur 75 solidaire par vissage du corps 71, par au moins une bille, telle que la bille 77, partiellement engagée dans une gorge du percuteur. Le dispositif de percussion comprend un poussoir 78 avec une gorge 79 et un ressort intérieur 76.

Le poussoir 78 coulisse sur l'extérieur du guide-percuteur 75 et il est retenu par des ergots 80 se déplaçant dans des rainures latérales 81. Ce poussoir 78 est ici l'organe de déclenchement.

Bien entendu pour initier la combustion du chargement pyrotechnique 72, sans sortir du cadre de l'invention, on peut utiliser des dispositifs d'initiation autre que le dispositif à percuteur ici décrit. Sans vouloir être exhaustif, nous citerons comme exemples des dispositifs d'initiation à pile électrique ou des dispositifs d'initiation piézo-électrique.

Eventuellement le générateur de gaz pyrotechnique peut être remplacé par un générateur de gaz constitué par un réservoir de gaz comprimé fermé par une vanne à ouverture rapide. L'organe de déclenchement va ouvrir ladite vanne, les gaz comprimés du réservoir vont se détendre et agir sur le moyen de poussée.

Pour l'utilisation, après avoir enlevé le bouchon d'asepsie, et posé la face aval de l'injecteur sur la peau du sujet à traiter, l'opérateur appuie, avec son pouce, sur le poussoir 78 qui s'enfonce en comprimant le ressort 76. Le poussoir se déplace jusqu'à ce que la gorge 79 arrive à la hauteur de la gorge du percuteur 74, les billes, telle que la bille 77, retenant le percuteur 74, se dégagent dans la gorge 79 et libèrent le percuteur qui va impacter violemment l'amorce 73, dont l'initiation enflamme le chargement pyrotechnique 72. Le percuteur 74 en appui sur le porte-amorce 30 assure le maintien en place de l'amorce et l'étanchéité : les gaz de combustion ne remontant pas vers le poussoir.

La combustion du chargement pyrotechnique va produire des gaz qui agissent sur le piston 11.

La figure 1 représente une seringue, selon l'invention, en forme de stylo : tous les éléments ont le même axe central mais sont superposés. Sans sortir du cadre de la présente invention d'autres dispositions sont envisageables par exemple la partie moteur peut faire un certain angle avec la partie réservoir-réceptacle pour arriver à des formes plus compactes comme cela est décrit par exemple dans la demande de brevet FR 2 815 544.

La figure 2 est un agrandissement de la partie aval de la seringue précédemment décrite.

Le réceptacle 7 est une pièce de forme extérieure cylindroconique, emmanchée dans le corps 2 de la seringue.
L'alésage central borgne 10 est essentiellement cylindrique. Préférentiellement le bord amont, raccordant la face amont du réceptacle et l'alésage comporte un arrondi avec un rayon de courbure suffisant pour ne pas déchirer l'obturateur aval quand il s'engage dans l'alésage au début du fonctionnement.
Le réceptacle comporte plusieurs conduits d'injection dont un seul, repéré 8, est visible sur cette vue. Une entrée 9 relie l'alésage, plus précisément la partie amont dudit alésage au conduit d'injection. Quand l'obturateur aval est entièrement logé dans l'alésage de liquide qui vient occuper la partie amont peut s'écouler jusqu'au conduit d'injection.
On voit en coupe un joint multilobé 12 placé dans une gorge de joint qui est au plus près des bords de l'alésage et contourne le lamage. Ce point sera décrit plus en détail par la suite.
Les figures 3 et 4 représentent une autre réalisation d'un réceptacle d'une seringue selon l'invention. Dans cet exemple le réceptacle est obtenu par injection d'un polycarbonate compatible avec l'uti-lisation envisagée.
Ledit réceptacle 37 comporte sur sa face latérale un filetage latéral pour faire le montage par vissage dans un corps de forme appropriée. Le réceptacle comporte trois conduits périphériques d'injection dont un seul 38 est visible sur la vue en coupe. Les trois conduits d'injection sont, dans cet exemple, identiques et équirépartis autour de l'alésage central 31 du réceptacle, ils sont reliés audit alésage par des entrées 39. Compte tenu du mode de fabrication, par injection, du réceptacle la partie amont du conduit est assez large, environ 0,8mm de diamètre sur environ 4mm à environ 5mm de hauteur, cette portion est cylindrique ou cylindroconique ; elle se prolonge vers l'aval par une partie plus étroite d'environ 0,1mm de diamètre et de environ 2mm à environ 3mm de hauteur pour réaliser le jet qui va pénétrer plus ou moins profondément dans la peau du patient.
Dans cette réalisation la face amont du réceptacle comporte un dispositif de clipsage 40 qui va recevoir et maintenir un réservoir comportant des bourrelets à ses extrémités ; ledit réservoir peut être prérempli avant le montage sur le réceptacle ; cet ensemble autonome constitue dans cet exemple la partie pharmaceutique de la seringue, elle peut être manipulée comme un tout. Ce dispositif est décrit plus en détail par ailleurs.
La face amont du réceptacle comporte un joint trilobé 34 puisqu'il y a trois conduits d'injection dans cet exemple. Ce joint est fabriqué par biinjection. Le joint trilobé est parallèle et au plus près des bords de l'alésage et des bords des entrées dans le sens où nous l'avons précédemment défini.
La vue en coupe montre que l'empreinte recevant le joint trilobé est de section rectangulaire. Le joint trilobé occupe toute cette empreinte avec une surépaisseur 35 sur la partie centrale de sa face amont libre, puisque le réservoir n'est pas encore monté sur le réceptacle.
Pour fixer encore davantage le joint trilobé dans l'empreinte du réceptacle la face aval du joint comporte des picots d'ancrage équirépartis qui se logent dans des orifices préparés dans l'empreinte au moment de l'injection du réceptacle. Un tel picot 36 est visible à l'opposé du conduit d'injection.
L'élastomère choisi pour biinjecter le joint doit convenablement adhérer à son support : c'est l'un des intérêts de la technique de biinjection ; il doit aussi être apte à un vieillissement prolongé et sa dureté doit être suffisante pour assurer la fonction d'étanchéité. Une dureté shore d'environ 75 convient pour cette appli-cation. Lorsque le réceptacle est en polycarbonate, l'élastomère du joint peut être du SANTOPRENE®.
Dans cet exemple l'alésage central borgne est de forme tronconique avec un angle d'environ 7°.

Le diamètre d'entrée c'est-à-dire l'amont du réceptacle de l'alésage est égal au diamètre du réservoir.
Les bords de l'alésage : intersection de la face amont du réceptacle et partie tronconique de l'alésage ne se coupent pas en formant une arête vive mais ces faces sont raccordées avec une courbure convenable que l'on voit sur la figure 4.
De même les bords du canal radial : intersection du canal radial et de l'alésage sont arrondis, avec un rayon de courbure plus petit que pour le cas précédent.

Les figures 5, 6 et 7 schématisent, vues partiellement en perspective, différentes réalisations de disposition d'entrée dans un conduit d'injection d'un réceptacle selon l'invention. Pour ces figures les parties analogues sont désignées par les mêmes repères que sur les figures 3 et 4.
La figure 5 présente une première réalisation d'une entrée 39 avec un lamage 59 centré sur le conduit d'injection 38 et un canal radial de largeur constante reliant le lamage et l'alésage central. Dans cet exemple les côtés du lamage et du canal sont perpendiculaires à la face amont du réceptacle et au fond 19 de l'entrée 39.
La figure 6 présente une autre réalisation qui diffère de la précédente par le canal qui est plus large au droit de l'alésage et qui converge en quelque sorte pour se raccorder au lamage 59 centré sur le conduit d'injection 38.
La figure 7 présente une réalisation avec un canal de largeur constante, mais les côtés du canal et du lamage 59 ne sont pas perpendiculaires mais sont arrondis pour ce raccorder avec un grand rayon de courbure à la zone du fond 19 du canal et du lamage, c'est un dispositif d'entrée de section profilée.

Sur ces trois schémas, par mesure de simplifications, les intersections d'une part entre l'alésage et la face amont du réceptacle et d'autre part l'alésage et les canaux radiaux sont représentés par des arcs de courbe ce qui laissent supposer des arêtes vives, en fait ces arêtes sont émoussées par une courbure suffisante pour les raccordements.

## Revendications

1. Seringue sans aiguille comportant un corps (2) logeant un réservoir cylindrique (3) fermé par un obturateur amont déplaçable (4) et un obturateur aval déplaçable (5) emprisonnant un principe actif (6) et comportant à l'aval un réceptacle (7,37) avec au moins deux conduits périphériques d'injection (8,38), ledit réceptacle en appui par sa face amont sur le réservoir et comprenant un alésage borgne (10,31), dont la hauteur libre permet le dégagement des entrées (9,39) des conduits périphériques (8,38) lorsque l'obturateur aval (5) est amené au contact du fond (7a) de l'alésage dudit réceptacle par le fonctionnement d'un moyen moteur (70) déplaçant l'ensemble obturateur amont-liquide-obturateur aval, ladite seringue étant **caractérisée en ce que** sur la face amont du réceptacle chaque entrée (9,39) comprend un lamage positionné sur le conduit , d'injection et est raccordé à un canal radial débouchant dans l'alésage central (10,31), et **en ce que** la face amont du réceptacle comporte un joint multilobé (12,34) parallèle au plus près du bord de l'alésage (10,31) et contournant au plus près les entrées (9,39) des conduits d'injection.

2. Seringue sans aiguille selon la revendication 1 **caractérisée en ce que** le dit joint multilobé (12) est logé dans une gorge de forme conjuguée.

3. Seringue sans aiguille selon la revendication 1 **caractérisée en ce que** la face amont du réceptacle comporte un joint multilobé (34) biinjecté parallèle au plus près du bord de l'alésage central et contournant au plus près les entrées des conduits d'injection.

4. Seringue sans aiguille selon l'une des revendications précédentes **caractérisée en ce que** le lamage centré sur le conduit d'injection a un diamètre compris entre environ 1,1 fois et 1,5 fois le diamètre du conduit d'injection et a une profondeur comprise entre environ 0,5 fois et environ 0,6 fois le diamètre du conduit d'injection.

5. Seringue sans aiguille selon la revendication 4 **caractérisée en ce que** le canal radial est de largeur constante et égale au diamètre du lamage.

6. Seringue sans aiguille selon la revendication 4 **caractérisée en ce que** le canal radial est de largeur croissante depuis son raccordement au lamage jusqu'à son débouché dans l'alésage central borgne où sa largeur est au plus égale 1,4 fois le diamètre du lamage.

7. Seringue sans aiguille selon l'une des revendications 4, 5 ou 6 **caractérisée en ce que** la lamage et le canal radial sont profilés .

8. Seringue sans aiguille selon l'une des revendications précédentes **caractérisée en ce que** l'alésage central borgne (31) est de forme essentiellement tronconique, le diamètre d'entrée de l'alésage étant égal au diamètre intérieur du réservoir, le plus petit diamètre étant vers le fond de l'alésage et une génératrice courante de la partie latérale tronconique faisant un angle compris entre environ 2° et environ 9° avec l'axe de l'alésage.

9. Seringue sans aiguille selon la revendication 8 **caractérisée en ce que** les bords amonts de l'alésage central (31) et des entrées comportent des courbures pour ne pas déchirer l'obturateur aval.

## Claims

1. Needleless syringe having a body (2) housing a cylindrical reservoir (3) closed by a displaceable upstream obturator (4) and a displaceable downstream obturator (5) confining an active principle (6) and having, downstream, a receptacle (7, 37) with at least two peripheral injection conduits (8, 38), the said receptacle resting, by its upstream face, on the reservoir and comprising a blind bore (10, 31), the free height of which permits clearing of the inlets (9, 39) of the peripheral conduits (8, 38) when the downstream obturator (5) is brought into contact with the bottom (7a) of the bore in the said receptacle by the functioning of a driving means (70) displacing the upstream obturator/liquid/downstream obturator combination, the said syringe being **characterised in that**, on the upstream face of the receptacle, each inlet (9, 39) comprises a counterbore positioned on the injection conduit and is connected to a radial duct opening into the central bore (10, 31), and **in that** the upstream face of the receptacle has a multi-lobed seal (12, 34) which is parallel, as closely as possible, to the edge of the bore (10, 31) and skirts, as closely as possible, the inlets (9, 39) of the injection conduits.

2. Needleless syringe according to Claim 1, **characterised in that** the said multi-lobed seal (12) is housed in a channel which is paired in shape.

3. Needleless syringe according to Claim 1, **characterised in that** the upstream face of the receptacle has a bi-injected multi-lobed seal (34) which is parallel, as closely as possible, to the edge of the central bore and skirts, as closely as possible, the inlets of the injection conduits.

4. Needleless syringe according to one of the preceding claims, **characterised in that** the counterbore centred on the injection conduit has a diameter of between about 1.1 times and 1.5 times the diameter of the injection conduit and has a depth of between about 0.5 times and about 0.6 times the diameter of said injection conduit.

5. Needleless syringe according to Claim 4, **characterised in that** the radial duct has a width which is constant and equal to the diameter of the counterbore.

6. Needleless syringe according to Claim 4, **characterised in that** the radial duct has a width which increases from its connection to the counterbore to its outlet into the blind central bore, where its width is at most equal to 1.4 times the diameter of said counterbore.

7. Needleless syringe according to one of Claims 4, 5 or 6, **characterised in that** the counterbore and the radial duct are profiled.

8. Needleless syringe according to one of the preceding claims, **characterised in that** the blind central bore (31) is essentially frustoconical in shape, the inlet diameter of said bore being equal to the internal diameter of the reservoir, the smallest diameter being towards the bottom of said bore and a standard generatrix of the frustoconical lateral part forming an angle of between about 2° and about 9° with the axis of the bore.

9. Needleless syringe according to Claim 8, **characterised in that** the upstream edges of the central bore (31) and of the inlets have curvatures so as not to tear the downstream obturator.

## Patentansprüche

1. Spritze ohne Nadel, die ein Gehäuse (2) umfasst, in dem ein zylindrischer Behälter (3) gelagert ist, der durch ein verschiebbares hinteres Verschlussstück (4) und ein verschiebbares vorderes Verschlussstück (5) verschlossen ist, die einen Wirkstoff (6) einschließen, und die am vorderen Ende ein Aufnahmeteil (7, 37) mit mindestens zwei peripheren Injektionskanälen (8, 38) umfasst, wobei das Aufnahmeteil mit seiner hinteren Stirnseite an den Behälter anliegt und eine Sackbohrung (10, 31) aufweist, deren freie Höhe die Freilegung der Eingänge (9, 39) der peripheren Kanäle (8, 38) ermöglicht, wenn das vordere Verschlussstück (5) durch die Funktion eines Antriebsmittels (70), das die Baugruppe "hinteres Verschlussstück - Flüssigkeit - vorderes Verschlussstück" verschiebt, in Kontakt mit dem Boden (7a) der Bohrung des Aufnahmeteils gebracht wird, wobei die Spritze **dadurch gekennzeichnet ist, dass** an der hinteren Stirnseite des Aufnahmeteils jeder Eingang (9, 39) eine Senkung aufweist, die auf dem Injektionskanal positioniert ist und an einen in die zentrale Bohrung (10, 31) einmündenden radialen Kanal angeschlossen ist, und **dadurch**, dass die hintere Stirnseite des Aufnahmeteils eine Mehrlippendichtung (12, 34) umfasst, die zum Rand der Bohrung (10, 31) parallel und möglichst nahe an diesem angeordnet ist und die Eingänge (9, 39) der Injektionskanäle so nahe wie möglich umgibt.

2. Spritze ohne Nadel nach Anspruch 1, **dadurch gekennzeichnet, dass** die Mehrlippendichtung (12) in einer Auskehlung von angepasster Form gelagert ist.

3. Spritze ohne Nadel nach Anspruch 1, **dadurch gekennzeichnet, dass** die hintere Stirnseite des Aufnahmeteils eine durch Zweikomponenten-Spritzgießen hergestellte Mehrlippendichtung (34) umfasst, die zum Rand der zentralen Bohrung parallel und möglichst nahe an diesem angeordnet ist und die Eingänge der Injektionskanäle so nahe wie möglich umgibt.

4. Spritze ohne Nadel nach einem der vorhergehenden Ansprüche, **dadurch gekennzeichnet, dass** die bezüglich des Injektionskanals zentrierte Senkung einen Durchmesser aufweist, der zwischen ungefähr dem 1,1-fachen und dem 1,5-fachen des Durchmessers des Injektionskanals beträgt, und eine Tiefe aufweist, die zwischen ungefähr dem 0,5-fachen und ungefähr dem 0,6-fachen des Durchmessers des Injektionskanals beträgt.

5. Spritze ohne Nadel nach Anspruch 4, **dadurch gekennzeichnet, dass** der radiale Kanal von einer konstanten Breite ist, die gleich dem Durchmesser der Senkung ist.

6. Spritze ohne Nadel nach Anspruch 4, **dadurch gekennzeichnet, dass** der radiale Kanal von einer Breite ist, die von seinem Anschluss an die Senkung bis zu seiner Einmündung in die zentrale Sackbohrung zunimmt, wobei seine Breite höchstens gleich dem 1,4-fachen des Durchmessers der Senkung ist.

7. Spritze ohne Nadel nach einem der Ansprüche 4, 5 oder 6, **dadurch gekennzeichnet, dass** die Senkung und der radiale Kanal profiliert sind.

8. Spritze ohne Nadel nach einem der vorhergehenden Ansprüche, **dadurch gekennzeichnet, dass** die zentrale Sackbohrung (31) im Wesentlichen kegelstumpfförmig ist, wobei der Eingangsdurchmesser der Bohrung gleich dem Innendurchmesser des Behälters ist, wobei der kleinste Durchmesser in der Nähe des Bodens der Bohrung vorhanden ist und eine gängige Erzeugende des kegelstumpfförmigen seitlichen Teils einen zwischen ungefähr 2° und ungefähr 9° betragenden Winkel mit der Achse der Bohrung einschließt.

9. Spritze ohne Nadel nach Anspruch 8, **dadurch gekennzeichnet, dass** die hinteren Ränder der zentralen Bohrung (31) und der Eingänge Krümmungen aufweisen, um das vordere Verschlussstück nicht zu zerreißen.
